# EUROPEAN PATENT APPLICATION

(11) **EP 4 706 592 A1**
(43) Date of publication of application: **11.03.2026**
(21) Application number: 25192432.0
(22) Date of filing: 29.07.2025
(51) Int. Cl.: A61F 2/04

(54) **IMPLANT**

(30) Priority: 06.09.2024 DE 102024125625
(71) Applicant: Olympus Winter & Ibe GmbH, 22045 Hamburg (DE)
(72) Inventor: Herzog, Eva, 53229 Bonn (DE); Wechsler, Wladislav, 10115 Berlin (DE)
(74) Representative: Hoener, Matthias

(57) **Abstract**

The invention creates an implant with which a bladder neck can be treated in a simple and reliable manner. This is achieved by an implant, in particular a removable implant, having a pressure means structure for expanding the bladder neck of a person. This pressure means structure exerts local pressure on the tissue of the bladder neck. The pressure means structure can be connected to a holding means for handling. This holding means, which may be a wire or a thread or a rod, can be used to insert the implant or the pressure means structure through the urethra into the bladder neck and, if necessary, to retrieve the pressure means structure from the patient's body after the end of the treatment.

## Description

The invention relates to an implant according to claim 1.

Various methods and techniques are known for the treatment of the urinary tract, in particular benign prostatic hyperplasia (BPH). In a minimally invasive treatment of BPH symptoms that is particularly gentle on the body, a removable implant is temporarily placed in the urethra or in the prostatic part of the patient's urethra. Such an implant is a wire structure made of a shape memory alloy such as nitinol. In a folded state, the wire structure is pushed through a catheter into the correct position, where it unfolds into its predetermined basic structure. This structure, which can be formed from three or four wires, is a basket structure. This basket structure expands the urethra. Due to the expansion of the wire structure against the urethral tissue, the urethral tissue is denatured over the course of a few days. This denaturation of the tissue occurs due to ischemic pressure of the individual wires on the cells of the tissue, which leads to reduced or completely absent blood flow. As a result, the lack of blood flow leads to a lack of oxygen in the cells and ultimately to cell death. Within a few days, the tissue can be reduced to such an extent that the urine flow is almost normalized. At the end of the treatment, the implant can be removed from the urethra using a catheter. Further details on such implants are described, for example, in DE 10 2021 133 394 A1.

The implant described above can also be used to treat an at least partially closed or blocked bladder neck, but this treatment is more of a side effect of the treatment of BPH symptoms, which is why its effectiveness is very limited. Since an elevated bladder neck in particular results from an overgrowth of the posterior lobe or the middle lobe and the prostate, this symptom cannot be treated by manipulating the tissue as described above. In fact, the use of the implant described above to treat the bladder neck can also be detrimental. In particular, the use of the three wires, which are arranged at a distance of 120° from each other, and the resulting highly localized pressure on the tissue are not suitable for the treatment of a constricted bladder neck.

On this basis, the invention is based on the problem of creating an implant with which a bladder neck can be treated in a simple and reliable manner.

A solution to this problem is described by the features of claim 1. Accordingly, it is provided that an implant, in particular a removable implant, has a pressure means structure for expanding the bladder neck of a person. This pressure means structure exerts a local pressure on the tissue of the bladder neck. The pressure means structure can be connected to a holding means for handling. This holding means, which may be a wire or a thread or a rod, can be used to insert the implant or the pressure means structure through the urethra into the bladder neck and, if necessary, to retrieve the pressure means structure from the patient's body after the end of the treatment.

An essential feature of the invention is that the pressure means structure has at least one plane or flat pressure means. The implant can exert a localized but large-area pressure on the tissue of the bladder neck by means of this plane pressure means. At a given force, this plane pressure means structure only leads to a low surface pressure that does not correspond to the ischemic pressure, which is why the tissue of the flap in question is not deformed or denatured. At the same time, however, the implant can act on the posterior part of the bladder neck and on the middle lobe, which can counteract the narrowing of the bladder neck.

It is preferable for one surface of the pressure means to be convex or concave. Depending on the application or orientation of the plane, in particular plate-like pressure means, it can be advantageous if the surface that comes into contact with the fabric is concave or convex. Due to the special curvature of the surface, the fabric can lie particularly well in the pressure means structure, whereby a particularly advantageous pressure builds up. The use of the claimed implant is particularly efficient due to the even distribution of pressure. However, it is also conceivable that the surface has a much more complex shape, such as a hyperbolic shape.

It is also possible for the plane pressure means to have a closed or at least partially open, preferably mesh-like, surface. It is also conceivable that the surface is designed as a grid of parallel or intersecting wires. These variations allow different effects or pressures to be exerted on the surrounding tissue.

Preferably, it is conceivable that the pressure means is shaped like a shovel and serves to support edge areas of the bladder neck, in particular a rear part of the bladder neck and/or a middle lobe.

A preferred embodiment of the invention may provide that a length of the pressure means is 0.5 mm to 30 mm. Furthermore, it is conceivable that a width of the pressure means is 1 mm to 10 mm or that a width of the pressure means increases continuously from a first end to a second end. It is also conceivable that a radius of curvature of the curved surface of the pressure medium is 1 mm to 20 mm. Finally, it is conceivable that several different implants of different geometries of the pressure medium are available to the surgeon in order to be able to react appropriately to the respective situation and to be able to apply the most homogeneous pressure possible to the affected tissue.

In particular, it is conceivable that a cross-section of the pressure means is wedge-shaped, whereby a base of the wedge is convex. Preferably, the base represents a closed surface and the wedge shape is formed by a wire structure. This special design can ensure the positioning of the implant within the bladder neck. At the same time, it is possible for the flat base to exert a rather large but low pressure on the tissue and for the wire structure to exert a highly localized, higher pressure on an opposing tissue. This combination of different pressure surfaces allows different tissue treatments to be carried out with one implant.

Another possible embodiment of the implant may provide for a cross-section of the pressure means to be arcuate, with an apex region of the arc representing a closed surface. The shape of the arch, on the other hand, can be formed by a wire structure. This embodiment also makes it possible to exert a more homogeneous and lower pressure on the fabric through the flat apex area and a highly localized high pressure through the wire structure. In addition, a large number of other embodiments are conceivable, which can exert an almost customized pressure on the tissue to be treated due to their special shapes.

A particularly advantageous embodiment of the invention may provide for a preferably sheet-like anchoring clamp to be arranged on the retaining means or the pressure means. This anchoring clamp can protrude into the bladder and, due to its curved structure, acts as a kind of clamp or return spring which protects the implant against slipping into the bladder neck. It is conceivable that this clamp is inserted through a tube into the urethra and is only pushed out of this tube at the correct position and can thus be positioned with pinpoint accuracy. Similarly, this clamp can be pulled back into the tube at the end of the treatment. It is also conceivable that the clamp can be removed from the bladder neck by turning or other movement. This anchoring clamp allows the treatment to be carried out with particular precision and reliability using the implant described.

According to the invention, it can be provided that the pressure means can be folded and unfolded from the folded state into an active state. For example, the implant can be pushed through the urethra and in particular through a tube by means of the holding means, which is designed as a tube, wire or thread, and placed in the correct position. As soon as the pressure means is in the correct position, it can unfold. Once the treatment is complete, the pressure means structure can be removed from the bladder neck in reverse order. For particularly special applications, it is also possible for the implant to remain in the body.

Furthermore, according to the invention, it may be provided that the at least one holding means and/or the pressure means is made of a metal, a plastic or a biodegradable material or a material with a shape memory, in particular nitinol. The embodiment made of a biodegradable material is particularly advantageous, since this material does not have to be salvaged but decomposes after some time.

Finally, a particularly preferred embodiment of the invention is that the surface of the printing medium is provided with a coating for the absorption and, preferably dosed, release of a medicament to an environment. This coating can be designed as a reservoir which can be filled with the corresponding medication prior to treatment. This reservoir can, for example, be a fire, a foam or another material through which the medication can be dispensed into the environment in doses. In this way, the medication can be dosed and used in a very targeted manner. The use of such a coating means that the implant has a dual effect. Firstly, the pressure is exerted on the surrounding tissue and secondly, the medication is applied with pinpoint accuracy.

A preferred embodiment of the present invention is explained in more detail below with reference to the drawing. This shows:
Fig. 1 A schematic representation of a possible embodiment of an implant,
Fig. 2 a schematic representation of a further embodiment of an implant,
Fig. 3 a perspective view of a further embodiment of an implant,
Fig. 4 a perspective view of a further embodiment of an implant, and
Fig. 5 a perspective view of a further embodiment of an implant.

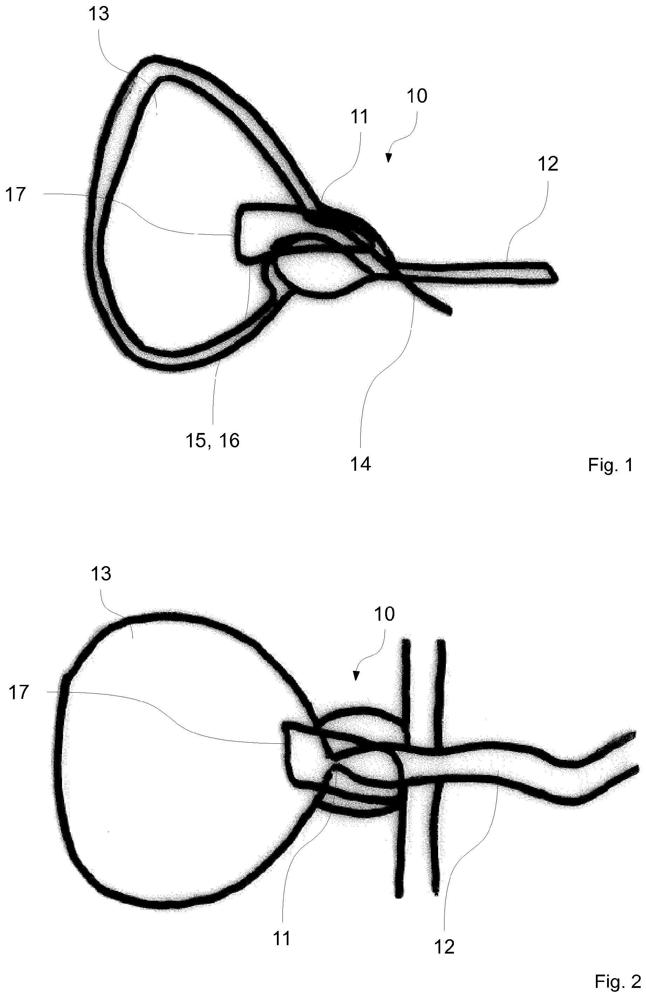

Fig. 1 shows a highly schematized representation of a possible embodiment of an implant 10. The implant 10 shown in Fig. 1 is used for a raised bladder neck 11. For this symptom, the implant 10 is pushed through a patient's urethra 12 into the bladder neck 11 so that part of the implant 10 protrudes into the bladder 13. The implant 10 essentially consists of a holding means 14 and a pressure means structure 15. The pressure means structure 15 is pushed through the urethra 12 together with the holding means 14, which may be a thread, a tube, wire or a rod. The holding means 14 can be used to maneuver the pressure means structure 15 into the correct position in the bladder neck 11 and secure it if necessary. At the end of the treatment, the pressure means structure 15 can be retrieved or pulled out of the bladder neck 11 via the holding means 14.

The pressure means structure 15 comprises a pressure means 16. This pressure means 16 is plane and presses with a surface 17 on the fabric to be treated. It is conceivable that the surface 17 of the pressure means is concave or convex. The aim of using the pressure means structure 15 is to exert a two-dimensional pressure on the fabric that does not denature the fabric, for example by using a wire. Depending on the circumstances or application and the design of the fabric to be treated, it may be advantageous for the surface 17 to be concave or convex in order to achieve the best possible surface contact.

Preferably, the length of the pressure means 16 is 0.5 mm to 20 mm and has a width of 1 mm to 5 mm. However, it is also conceivable that the pressure means 16 is fan-shaped and that its width increases continuously starting from the holding means 14.

It is envisaged that the holding means 14 and/or the pressure means 16 are made of a metal, a plastic or a biodegradable material or a material with a shape memory, such as nitinol. In particular, a biodegradable implant 10 has the advantage that it dissolves over time and does not have to be removed from the body. An implant 10, in particular a pressure medium 16, made of a shape memory material can also prove to be advantageous, as the pressure medium 16 can be brought into position in a folded state and unfolds into its active form there. This folded insertion is particularly gentle on the person's body.

In the embodiment example shown in Fig. 2, the implant 10 or the pressure means structure 15 is used to treat a narrowed bladder neck 11. There, the pressure means 16 is positioned in the constriction so that the flat pressure means 16 acts on the surrounding tissue. Depending on the nature of the constriction, the curvature of the pressure means 16 can be adjusted or selected accordingly by the person being treated.

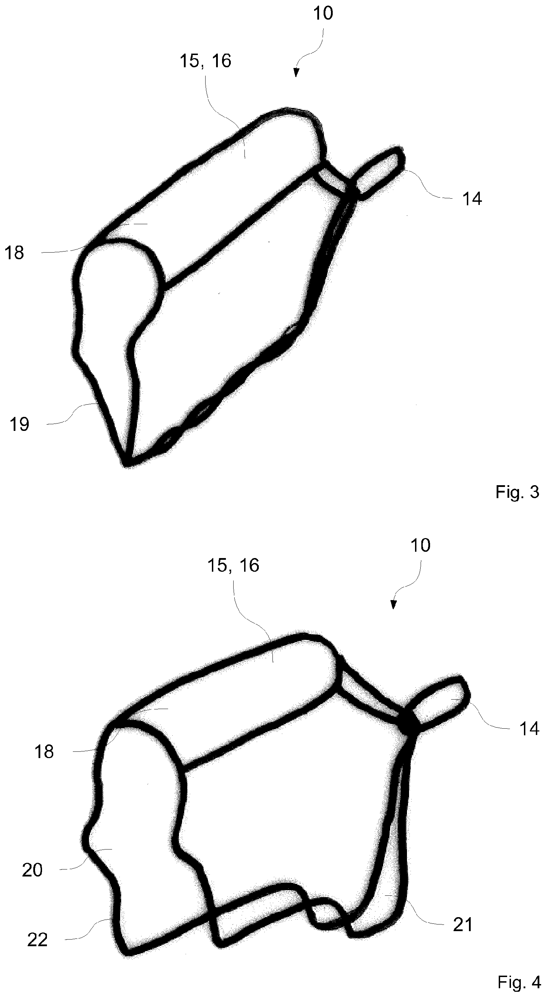

Fig. 3 shows a further embodiment example for an implant 10. In this embodiment example, the pressure means 16 has a wedge-shaped cross-section. This wedge-shaped form is formed by a base with a closed surface 18 and an open wire structure 19, through which legs of the wedge are formed. This embodiment example of the implant 10 also has a retaining means 14, as described above. The convex surface 18 exerts a homogeneous pressure on the surrounding tissue, whereas the wire structure 19 exerts a local pressure on the tissue, which results in the tissue being less well supplied with blood and thus denaturing. This particular embodiment of the implant 10 makes it possible to treat both a high bladder neck 11 and a narrowed bladder neck 11.

A further embodiment example for an implant 10 is outlined in Fig. 4. This embodiment example also has a convex surface 18, to which, however, two parallel or at least almost parallel sides 20, 21 are connected, so that a cross-section of the surface 18 and the sides 20, 21 form an arc. The sides 20, 21 are also formed by a wire structure 22, which exerts a similar effect on the surrounding tissue as previously described for the embodiment example according to Fig. 3. This embodiment example for an implant 10 according to Fig. 4 can also exert a flat and homogeneous pressure on the tissue via the surface 18.

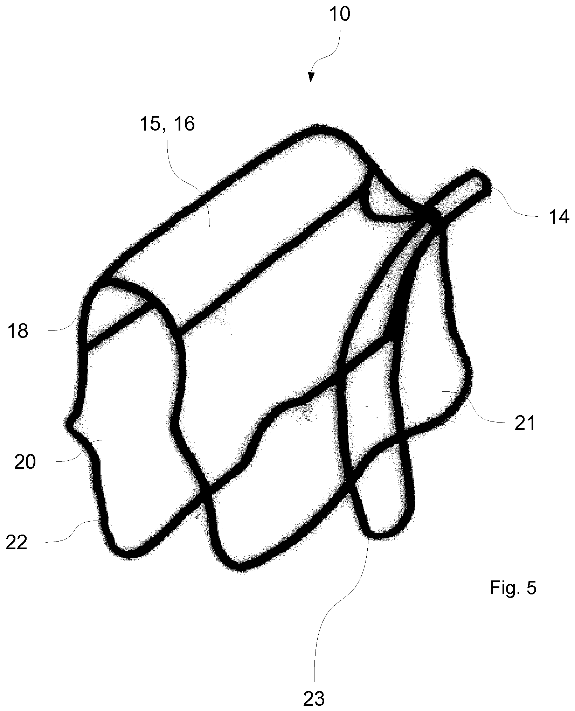

In order to fix the implant 10 in position in the bladder neck 11, it may be provided that the pressure medium structure 15 has an anchoring clamp 23. In Fig. 5, an example of an anchoring clamp 23 is shown using the embodiment example of an implant 10 according to Fig. 4. This anchoring clamp 23 is designed like a tab and has at least a low spring force, so that it serves as an anchoring or anchoring means after insertion into the bladder neck 11. The anchoring clamp 23 protrudes into the bladder neck 11 and also presses the surface 18 against the opposite tissue of the bladder neck 11. After completion of the treatment, the anchoring clamp 23 can be removed or pulled out of the bladder neck 11. Similarly, it is conceivable that the other embodiments of an implant 10 shown here also have an anchoring as shown in Fig. 5.

### List of Reference Numerals:

- 10: Implant
- 11: Bladder neck
- 12: Urethra
- 13: Bladder
- 14: Holding means
- 15: Pressure means structure
- 16: Pressure means
- 17: Surface
- 18: Area
- 19: Wire structure
- 20: Side
- 21: Side
- 22: Wire structure
- 23: Anchoring clamp

## Claims

1. Implant (10), in particular a removable implant, with a pressure means structure (15) attached to a holding means (14) for expanding the bladder neck (11) of a person by applying local pressure to the tissue of the bladder neck (11), **characterized in that** the pressure means structure (15) has at least one plane pressure means (16).

2. Implant (10) according to claim 1, **characterized in that** a surface (17) of the pressure means (16) is convex or concave.

3. Implant (10) according to claim 1 or 2, **characterized in that** the flat pressure means (16) has a closed or an at least partially open, preferably net-like, surface (17).

4. Implant (10) according to one of the preceding claims, **characterized in that** the pressure means (16) is designed in a blade-like manner and serves to support edge regions of the bladder neck (11), in particular a rear part of the bladder neck (11) and/or a middle lobe.

5. Implant (10) according to one of the preceding claims, **characterized in that** a length of the pressure means (16) is 0.5 mm to 30 mm.

6. Implant (10) according to one of the preceding claims, **characterized in that** a width of the pressure means (16) is 1 mm to 10 mm or that a width of the pressure means (16) increases continuously from a first to a second end.

7. Implant (10) according to one of the preceding claims, **characterized in that** a radius of curvature of the curved surface of the pressure means (16) is 1 mm to 20 mm.

8. Implant (10) according to one of the preceding claims, **characterized in that** a cross-section of the pressure means (16) is wedge-shaped, wherein a base of the wedge is convex.

9. Implant (10) according to claim 8, **characterized in that** the base is a closed area (18) and the wedge shape is formed by a wire structure (19).

10. Implant according to any one of the preceding claims, **characterized in that** a cross-section of the pressure means (16) is arcuate, wherein an apex region of the arc is a closed area (18) and the shape of the arc is formed by a wire structure (22).

11. Implant (10) according to one of the preceding claims, **characterized in that** the holding means (14) is designed as a tube, wire or thread.

12. Implant (10) according to one of the preceding claims, **characterized in that** an anchoring clamp (23), preferably in the form of a leaf, is arranged on the holding means (14) or the pressure means (16).

13. Implant (10) according to one of the preceding claims, **characterized in that** the pressure means (16) is foldable and unfolds from the folded state into an active state.

14. Implant (10) according to one of the preceding claims, **characterized in that** the at least one holding means (14) and/or the pressure means (16) are made of a metal, a plastic or a biodegradable material or a material with a shape memory, in particular nitinol.

15. Implant (10) according to one of the preceding claims, **characterized in that** the surface (17) of the pressure means (16) is formed with a coating for the absorption and, preferably dosed, release of a medicament to an environment.
